# EUROPEAN PATENT SPECIFICATION

(11) **EP 1 781 255 B1**
(45) Date of publication and mention of the grant of the patent: **15.02.2012**
(21) Application number: 05783719.7
(22) Date of filing: 09.08.2005
(51) Int. Cl.: A61K 9/127, A61K 31/475, A61K 31/573, A61P 35/00, A61P 35/02

(54) **COMPOSITIONS AND METHODS FOR TREATING LEUKEMIA**
ZUSAMMENSETZUNGEN UND VERFAHREN ZUR BEHANDLUNG VON LEUKÄMIE
COMPOSITIONS ET PROCEDES POUR TRAITER LA LEUCEMIE

(30) Priority: 10.08.2004 US 651482 P
(43) Date of publication of application: 09.05.2007
(73) Proprietor: Talon Therapeutics, Inc., San Mateo, CA 94404 (US)
(72) Inventor: THOMAS, Deborah, A., Pearland, Texas 77584 (US)
(74) Representative: Grünecker, Kinkeldey, Stockmair & Schwanhäusser
(86) International application number: PCT/US2005/028233
(87) International publication number: WO 2006/020618

(56) References cited:
- WO-A-00/59473
- US-A1- 2004 071 768
- THOMAS D A ET AL: "PHASE II STUDY OF LIPOSOMAL VINCRISTINE (LIPOV) IN RELAPSED OR REFRACTORY ADULT ACUTE LYMPHOBLASTIC LEUKEMIA (ALL)" BLOOD, W.B.SAUNDERS COMPANY, ORLANDO, FL, US, vol. 94, no. 10 SUPPL 1 PART 2, 15 November 1999 (1999-11-15), page 238B, XP009039110 ISSN: 0006-4971
- SARRIS A H ET AL: "LIPOSOMAL VINCRISTINE IN RELAPSED NON-HODGKIN'S LYMPHOMAS: EARLY RESULTS OF AN ONGOING PHASE II TRIAL" ANNALS OF ONCOLOGY, KLUWER, DORDRECHT, NL, vol. 11, no. 1, January 2000 (2000-01), pages 69-72, XP009039101 ISSN: 0923-7534
- BOSTROM BRUCE C ET AL: "Dexamethasone versus prednisone and daily oral versus weekly intravenous mercaptopurine for patients with standard-risk acute lymphoblastic leukemia: a report from the Children's Cancer Group." BLOOD. 15 MAY 2003, vol. 101, no. 10, 15 May 2003 (2003-05-15), pages 3809-3817, XP002356997 ISSN: 0006-4971
- KANTARJIAN H M ET AL: "Experience with vincristine, doxorubicin, and dexamethasone (VAD) chemotherapy in adults with refractory acute lymphocytic leukemia." CANCER. 1 JUL 1989, vol. 64, no. 1, 1 July 1989 (1989-07-01), pages 16-22, XP009058145 ISSN: 0008-543X
- KANTARJIAN H M ET AL: "Acute lymphocytic leukaemia in the elderly: characteristics and outcome with the vincristine-adriamycin-dexamethasone (VAD) regimen." BRITISH JOURNAL OF HAEMATOLOGY. SEP 1994, vol. 88, no. 1, September 1994 (1994-09), pages 94-100, XP009058146 ISSN: 0007-1048

## Description

### BACKGROUND OF THE INVENTION

### Field of the Invention

This invention relates to methods and compositions for the treatment of a neoplasia in a mammal and, in particular, to liposomal drug formulations for the treatment of leukemia.

### Description of the Related Art

Despite years of research into the development of new methods of treatment, hematologic malignancies are not well addressed by current treatment regimens. Further, leukemia and lymphoma affect a significant proportion of the population. For example, more than 60,000 people in the United States are diagnosed with lymphoma each year, including more than 55,000 cases of non-Hodgkin's Lymphoma (NHL), and these numbers are constantly increasing. In addition, the prognosis for those affected by these diseases is often poor, as the survival rates for leukemia and lymphoma patients remain low. Clearly, new methods for treating these diseases are needed.

While traditional treatments for lymphoma typically depend on the type of lymphoma as well as the medical history of the patient, first-line treatment for many lymphomas typically includes chemotherapy. Such chemotherapy will often entail the administration of a "cocktail" of compounds, *e.g.*, the formulation CHOP, which includes cyclophosphamide, doxorubicin, vincristine, and prednisone. In addition, certain first-line cancer treatments also include other forms of cancer therapy, such as radiation therapy.

Similarly, in patients with leukemia, such as acute lymphoblastic leukemia (ALL), which is also called acute lymphocytic leukemia, treatment typically includes combination chemotherapy. Combination regimens used in the treatment of ALL frequently include the agents L-asparaginase, vincristine, corticosteroids and anthracyclines. While combinations that include an anthracycline (*e.g.*, VAD, which includes vincristine, doxorubicin (Adriamycin), and dexamethasone) are reported to improve the complete response rate, cardiotoxicity remains a major concern, particularly in elderly ALL patients. Clearly, more effective agents and combinations that can provide high response rates without causing serious, life-threatening toxicities are needed.

In many cases, patients respond initially to such first-line treatments, but subsequently suffer a relapse, *i.e.*, a tumor reappears or resumes growing. Following one such relapse, patients are often treated with further chemotherapy, *e.g.*, with CHOP or with other formulations. In some cases, the patients are treated with other procedures such as bone marrow transplantation. Again, in many cases, patients initially respond to such additional treatments, but subsequently suffer another relapse. In general, the more relapses a patient suffers, the less agreement there is in the art concerning optimal subsequent treatment. In other cases, a patient fails to respond at all to a treatment, even initially, and is thus said to have a refractory cancer. In these cases, as well, little agreement exists in the art regarding optimal subsequent treatment.

Alkaloids isolated from the periwinkle plant (*Vinca rosea*), called "vinca alkaloids," have proven effective for first line treatment of many types of lymphomas, leukemia, and other cancers. One such vinca alkaloid, vincristine, is included in the common chemotherapeutic combination regimens CHOP and VAD. Vincristine, which depolymerizes microtubules and thereby inhibits cell proliferation, is administered in its free form in CHOP and VAD.

Liposome-encapsulated vincristine has been reported (*see*, *e.g.*, U.S. Patent 5,741,516, or U.S. Patent No. 5,714,163). In particular, these patents discuss the use of vincristine encapsulated in phosphatidylcholine, distearoylphosphatidylcholine, or sphingomyelin, in addition to cholesterol. The use of liposomal vincristine in the treatment of neoplasia, including lymphoma and leukemia, has also been generally described (*see*, *e.g.*, U.S. Patent 6,723,338).

In general, lipid-encapsulated drug formulations may provide advantages over traditional drug-delivery methods. For example, some lipid-based formulations provide longer half-lives *in vivo*, superior tissue targeting, and decreased toxicity. For certain cancers, including particular leukemias and lymphomas, these properties may be advantageous. Numerous methods have been described for the formulation of lipid-based drug delivery vehicles (*see*, *e.g.*, U.S. Patent 5,741,516). However, there remains a need in the art for liposomal drug formulations, and combination therapies comprising these liposomal drug formulations, for the treatment of many leukemias and lymphomas, including ALL.

### BRIEF SUMMARY OF THE INVENTION

It has now been discoverer that the liposome-encapsulated vinca alkaloid vincristine is especially efficacious in combination with dexamethasone for the treatment of leukemia. Provided herein, therefore, are compositions and methods for the treatment of leukemia.

In one embodiment, the present invention provides composition for use in a method for treating a leukemia in a mammal, the method comprising administering to the mammal a pharmaceutical composition comprising the liposome-encapsulated vinca alkaloid vincristine in combination with dexamethasone.

In various embodiments, the leukemia is acute lymphoblastic leukemia (ALL), acute myeloid leukemia, acute promyelocytic leukemia, chronic myeloid leukemia, chronic lymphocytic leukemia, hairy cell leukemia, or myelodysplastic syndrome.

The vinca alkaloid is vincristine. In other embodiments, the liposome comprises distearoylphosphatidylcholine or sphingomyelin. In another embodiment, the liposome further comprises cholesterol. In yet another related embodiment, the liposome comprises a pH gradient. In another embodiment, the pH at the interior of the liposomes is lower than the pH at the exterior.

In one particular embodiment, the mammal is a human. In one embodiment, the treatment is a first-line treatment. In another embodiment, the mammal has previously undergone at least one chemotherapy treatment. In a specific embodiment, the previous chemotherapy treatment comprised administration of a free-form vinca alkaloid, such as vincristine, vinblastine, vindesine, or vinorelbine. In other embodiments, the chemotherapy treatment included an anthracycline-containing combination therapy. In one such embodiment, the anthracycline was doxorubicin, idarubicin or daunorubicin. In another embodiment, the mammal has exhibited a partial or complete response to the chemotherapy prior to a relapse of the cancer. In another embodiment, the relapse is a second relapse.

In a further embodiment, the liposome-encapsulated vinca alkaloid is administered systemically by intravenous delivery. The liposome-encapsulated vincristine is co-administered with dexamethosone. In another embodiment, the liposome-encapsulated vinca alkaloid is co-administered with a prophylactic or therapeutic treatment for neurotoxicity, such as gabapentin (Neurotonin™).

In yet another embodiment, the liposome-encapsulated vinca alkaloid is administered to the mammal once every 7-21 days, while in related embodiments, the liposome-encapsulated vinca alkaloid is administered once every 7 days or once every 14 days. In another embodiment, the liposome-encapsulated vinca alkaloid is administered at a dosage falling within a range of about 1.4 to about 2.4 mg/m² or about 1.4 to about 1.8 mg/m². In a related embodiment, the liposome-encapsulated vinca alkaloid is administered at a dosage falling within a range of 1.4 to 2.4 mg/m² or 1.4 to 2.8 mg/m².

In certain embodiments, the present invention provides an improvement on conventional methods, of treating cancer. In particular, the present invention provides a composition for use in method for treating leukemia in a mammal, comprising administering the liposome-encapsulated vinca alkaloid vincristine to the mammal in combination with dexamethasone in the absence of an anthracycline.

Kits including the herein-described formulations, and for preparing the herein-described formulations, as well as instructions for their use are also included.

The present invention, also provides the use of the liposome-encapsulated vinca alkaloid vincristine in the preparation of a medicament for the treatment of a neoplasia, including leukemia in the absence of an anthracycline. In certain uses, the leukemia is acute lymphoblstic leukemia (ALL). In certain uses, the neoplasia is a relapsed, indolent, aggressive, or transformed neoplasia, e.g., non-Hodgkin's Lymphoma. In particular uses, the medicament is used as a first line treatment for a neoplasia. In other preferred uses, the vinca alkaloid is present in the medicament at a dosage, e.g., of about 1.4 to about 2.4 mg/m², about 1.4 to about 2.8 mg/m², 1.4 to 2.4 mg/m², or 1.4 to 2.8 mg/m² and is administered once every 7-21 days, preferably every 14 days or every 7 days.

In yet another embodiment, the invention provides a composition for use in a method of treating a relapsed cancer in a human, comprising administering to said human a pharmaceutical composition comprising the liposome-encapsulated vinca alkaloid in combination with a steroid, wherein said relapsed cancer is a leukemia. The vinca alkaloid is vincristine. In one embodiment, vincristine is administered at a dosage of between 1.4 to 2.4 mg/m² or between 1.4 to 2.8 mg/m². The steroid is dexamethasone. In yet another embodiment, said dexamethasone is administered at a dosage of between 25-75 mg and, in one embodiment, 40 mg. In certain embodiments, the liposome comprises sphingomyelin and cholesterol. In particular embodiments, the ratio of sphingomyelin to cholesterol is between 75/25 (mol% sphingomyelin/mol% cholesterol) and 50/50 (mol% sphingomyelin/mol% cholesterol) or is approximately 55/45 (mol% sphingomyelin/mol% cholesterol). In another embodiment, said leukemia is acute lymphoblastic leukemia (ALL).

In a related embodiment, the invention further provides a composition for use in a method of treating acute lymphoblastic leukemia (ALL) in a human, comprising coadministering to said human a pharmaceutical composition comprising the liposome-encapsulated vinca alkaloid in combination with a steroid in the absence of an anthracycline. The vinca alkaloid is vincristine. In certain embodiments, vincristine is administered at a dosage of between 1.4 to 2.4 mg/m² or between 1.4 to 2.8 mg/m². In other embdoiments, vincristine is administered at a dosage greater than 1.4 mg/m² or without dose-capping at 2.0 or 2.5 mg total dose. The steroid is dexamethasone. In yet another embodiment, said dexamethasone is administered at a dosage of between 25-75 mg and, in one embodiment, 40 mg. In certain embodiments, the liposome comprises sphingomyelin and cholesterol. In particular embodiments, the ratio of sphingomyelin to cholesterol is between 75/25 (mol% sphingomyelin/mol% cholesterol) and 50/50 (mol% sphingomyelin/mol% cholesterol) or is 55/45 (mol% sphingomyelin/mol% cholesterol).

The invention further provides a composition for use in a method of treating acute lymphoblastic leukemia in a human, comprising coadministering to said human a pharmaceutical composition comprising liposome-encapsulated vincristine with dexamethasone. In one particular embodiment, the liposome comprises sphingomyelin and cholesterol at a ratio of between 75/25 (mol% sphingomyelin/mol% cholesterol) and 50/50 (mol% sphingomyelin/mol% cholesterol) or is 55/45 (mol% sphingomyelin/mol% cholesterol). In another embodiment, the vincristine is administered at a dosage of between 1.4 to 2.4 mg/m² or between 1.4 to 2.8 mg/m². In other embodiments, the vincristine is administered at a dosage greater than 1.4 mg/m², or without dose-capping at 2.0 or 2.5 mg total dose. In yet another embodiment, said dexamethasone is administered at a dosage of between at 25-75 mg and, in one embodiment, 40 mg. In particular embodiments, the ALL is relapsed or refractory ALL. In other embodiments, the treatment is a first-line treatment. The invention provides a method which does not further comprise administration of an anthracycline.

### Definitions

"Neoplasia," as used herein, refers to any aberrant growth of cells, tumors, malignant effusions, warts, polyps, nonsolid tumors, cysts and other growths. A site of neoplasia can contain a variety of cell types, including but not limited, to neoplastic cells, vascular endothelia, or immune system cells, such as macrophages and leukocytes, etc.

A "cancer" in a mammal refers to any of a number of conditions caused by the abnormal, uncontrolled growth of cells. Cells capable of causing cancer, called "cancer cells", possess a number of characteristic properties such as uncontrolled proliferation, immortality, metastatic potential, rapid growth and proliferation rate, and certain typical morphological features. Often, cancer cells will be in the form of a tumor, but such cells may also exist alone within a mammal, or may be a non-tumorigenic cancer cell, such as a leukemia cell. A cancer can be detected in any of a number of ways, including, but not limited to, detecting the presence of a tumor or tumors (*e.g.*, by clinical or radiological means), examining cells within a tumor or from another biological sample (e.g., from a tissue biopsy), measuring blood markers indicative of cancer (*e.g.*, CA125, PAP, PSA, CEA, AFP, HCG, CA 19-9, CA 15-3, CA 27-29, LDH, NSE, and others), and detecting a genotype indicative of a cancer (*e.g.*, TP53, ATM, *etc.*). However, a negative result in one or more of the above detection methods does not necessarily indicate the absence of cancer, *e.g.*, a patient who has exhibited a complete response to a cancer treatment may still have a cancer, as evidenced by a subsequent relapse.

"Systemic delivery," as used herein, refers to delivery that leads to a broad bio-distribution of a compound within an organism. Systemic delivery means that a useful, preferably therapeutic, amount of a compound is exposed to most parts of the body. To obtain broad bio-distribution generally requires a route of introduction such that the compound is not rapidly degraded or cleared (such as by first pass organs (liver, lung, *etc.*) or by rapid, nonspecific cell binding) before reaching a disease site. Systemic delivery of liposome-encapsulated vinca alkaloids is preferably obtained by intravenous delivery.

"Lymphoma" refers to a malignant growth of B or T cells in the lymphatic system. "Lymphoma" includes numerous types of malignant growths, including Hodgkin's Lymphoma and non-Hodgkin's lymphoma (NHL).

"Non-Hodgkin's Lymphoma" refers to a malignant growth of B or T cells in the lymphatic system that is not a Hodgkin's Lymphoma (which is characterized, *e.g.*, by the presence of Reed-Sternberg cells in the cancerous area). Non-Hodgkin's lymphomas encompass over 29 types of lymphoma, the distinctions between which are based on the type of cancer cells. The particular classification depends on the particular system of classification used, such as the Working formulation, the Rappaport classification, and the REAL classification. In preferred embodiments, the REAL classification is used.

"Leukemia" refers to a malignant growth of white blood cells in the bone marrow and/or blood. There are three major types of leukemias: (1) Acute lymphocytic leukemia (ALL), which is characterized by immature forms of lymphoid white blood cells in the bone marrow and is the most common childhood cancer; (2) Acute myelogenous leukemia (AML), wherein the bone marrow contains immature cells of the myeloid type. There are an estimated 10,000 new cases annually (Acute promyelocytic leukemia, or APL, is a subtype of AML); (3) Chronic myelogenous leukemia (CML), which is a moderately progressive form of leukemia that is characterized by the presence of large numbers of granulocytes in the blood.

A "relapsed cancer," leukemia or lymphoma refers to a cancer or lymphoma that has recurred following prior complete or partial remission in response to a prior treatment. Recurrence can be defined in any way, including a reappearance or re-growth of a tumor as detected by clinical, radiological, or biochemical assays, or by an increased level of a cancer marker. Prior treatments can include, but are not limited to, chemotherapy, radiation therapy, and bone marrow transplantation.

An "indolent" non-Hodgkin's Lymphoma is a classification that includes slow growing forms of lymphoma. They encompass what are called low grade and some categories of intermediate grade NHL in the Working Formulation. Indolent NHLs are sometimes not responsive to conventional cancer therapies such as chemotherapy and radiation therapy.

A "transformed" non-Hodgkin's Lymphoma is a classification sometimes employed to describe an indolent NHL which acquires an aggressive aspect and becomes more responsive to standard chemotherapies.

Patients with "refractory cancer" or "refractory lymphoma" are those who have failed to achieve complete remission on their first course of combination chemotherapy, or patients who have failed to achieve complete or partial remission on subsequent chemotherapy. "Primary refractory" patients are those who have never achieved complete remission even at first treatment.

A "stable disease" is a state wherein a therapy causes cessation of growth or prevalence of a tumor or tumors as measured by the usual clinical, radiological and biochemical means, although there is no regression or decrease in the size or prevalence of the tumor or tumors, *i.e.*, cancer that is not decreasing or increasing in extent or severity.

"Partial response" or "partial remission" refers to the amelioration of a cancerous state, as measured by tumor size and/or cancer marker levels, in response to a treatment. Typically, a "partial response" means that a tumor or tumor-indicating blood marker has decreased in size or level by about 50% in response to a treatment. The treatment can be any treatment directed against cancer, but typically includes chemotherapy, radiation therapy, hormone therapy, surgery, cell or bone marrow transplantation, immunotherapy, and others. The size of a tumor can be detected by clinical or by radiological means. Tumor-indicating markers can be detected by means well known to those of skill, *e.g.*, ELISA or other antibody-based tests.

A "complete response" or "complete remission" means that a cancerous state, as measured by, for example, tumor size and/or cancer marker levels, has disappeared following a treatment such as chemotherapy, radiation therapy, hormone therapy, surgery, cell or bone marrow transplantation, or immunotherapy. The presence of a tumor can be detected by clinical or by radiological means. Tumor-indicating markers can be detected by means well known to those of skill, *e.g.*, ELISA or other antibody-based tests. A "complete response" does not necessarily indicate that the cancer has been cured, however, as a complete response can be followed by a relapse.

"Chemotherapy" refers to the administration of chemical agents that inhibit the growth, proliferation and/or survival of cancer cells. Such chemical agents are often directed to intracellular processes necessary for cell growth or division, and are thus particularly effective against cancerous cells, which generally grow and divide rapidly. For example, vincristine depolymerizes microtubules, and thus inhibits cells from entering mitosis. In general, chemotherapy can include any chemical agent that inhibits, or is designed to inhibit, a cancerous cell or a cell likely to become cancerous. Such agents are often administered, and are often most effective, in combination, *e.g.*, in the formulation CHOP.

"Radiation therapy" refers to the administration of radioactivity to an animal with cancer. Radiation kills or inhibits the growth of dividing cells, such as cancer cells.

"Surgery" is the direct removal or ablation of cells, *e.g.*, cancer cells, from an animal. Most often, the cancer cells will be in the form of a tumor (*e.g.*, resulting from a lymphoma), which is removed from the animal.

"Hormone therapy" refers to the administration of compounds that counteract or inhibit hormones, such as estrogen or androgen, that have a mitogenic effect on cells. Often, these hormones act to increase the cancerous properties of cancer cells *in vivo*.

"Immunotherapy" refers to methods of enhancing the ability of an animal's immune system to destroy cancer cells within the animal.

A "free-form" therapeutic agent, or "free" therapeutic agent, refers to a therapeutic agent that is not liposome-encapsulated. Usually, a drug is presumed to be "free, or in a "free-form," unless specified otherwise. A vinca alkaloid in free form may still be present in combination with other reagents, however, such as other chemotherapeutic compounds, a pharmaceutical carrier, or complexing agents, *i.e.* as used herein the term only specifically excludes lipid formulations of the vinca alkaloids.

### DETAILED DESCRIPTION OF THE INVENTION

This invention provides methods of treating neoplasia in a patient. This invention is based on the surprising discovery that liposome-encapsulated vinca alkaloids are unexpectedly well tolerated and unusually effective in combination with dexamethasone. In particular, the surprising discovery was made that liposome-encapsulated vincristine can be administered at high doses on a weekly schedule in combination with dexamethasone without inducing severe toxicities. In one embodiment, vincristine, encapsulated in a sphingomyelin and cholesterol based liposome, is used in the treatment of leukemia, especially acute lymphoblastic leukemia (ALL). Accordingly, the invention provides, *inter alia*, methods of treating leukemias.

Liposome encapsulated vinca alkaloids in combination with dexamethasone can be used in first line treatment of leukemia or for the treatment of patients who have relapsed after previous leukemia therapy.

The present invention further provides dosages and dose scheduling of liposomal vinca alkaloids in combination with dexamethasone for treatment of leukemia.

### Cancers Treatable with Lipid-Encapsulated Vinca Alkaloids

The methods described herein can be used to treat any type of cancer. For example, these methods are applied to cancers of the blood and lymphatic systems, including lymphomas, leukemia, and myelomas.

In preferred embodiments, the present methods are used to treat any of the large number of leukemias. For example, acute lymphoblastic leukemia (ALL), acute promyelocytic leukemia, acute myeloid leukemia, chronic myeloid leukemia, chronic lymphocytic leukemia, hairy cell leukemia and myelodysplastic syndrome can be treated using the methods described herein. Indeed, any acute, chronic, myelogenous, and lymphocytic form of the disease can be treated using the methods of the present invention. The methods described herein are also applied to any form of leukemia, including adult and childhood forms of the disease. In particular embodiments, the methods are used to treat ALL in adult or pediatric patients. Other types of leukemia that can be treated according to the methods of the present invention include those described by the Leukemia Society of America at www.leukemia.org.

In addition, the methods and compositions described herein have application in the treatment of lymphomas. Such lymphomas include, but are not limited to, low-grade, intermediate-grade, and high-grade lymphomas, as well as both B-cell and T-cell lymphomas. Included in these categories are the various types of small cell, large cell, cleaved cell, lymphocytic, follicular, diffuse, Burkitt's, Mantle cell, NK cell, CNS, AIDS-related, lymphoblastic, adult lymphoblastic, indolent, aggressive, transformed and other types of lymphomas. The methods of the present invention can be used for adult or childhood forms of lymphoma, as well as lymphomas at any stage, e.g., stage I, II, III, or IV. The various types of lymphomas are well known to those of skill, and are described, e.g., by the American Cancer Society (see, e.g., www3.cancer.org).

Acute lymphoblastic leukemia (ALL) is the most common malignancy of childhood, representing nearly one third of all pediatric cancers, although ALL also occurs in adults. ALL may be called by several names, including acute lymphoid leukemia and acute lymphoblastic leukemia. Annual incidence of ALL is about 30 cases per million population, with a peak incidence in patients aged 2-5 years. Although a small percentage of cases are associated with inherited genetic syndromes, the cause of ALL remains largely unknown. While not wishing to be bound to any particular theory, it is believed that in many cases of ALL, a lymphoid progenitor cell becomes genetically altered and subsequently undergoes dysregulated proliferation and clonal expansion. In most cases, the pathophysiology of transformed lymphoid cells reflects the altered expression of genes whose products contribute to the normal development of B cells and T cells. ALL generally is thought to arise in the bone marrow, but leukemic blasts may be present systemically at the time of presentation, including in the bone marrow, thymus, liver, spleen, lymph nodes, testes, and the central nervous system (CNS).

ALL can develop from primitive lymphocytes that are in various stages of development, resulting in different subtypes of ALL. The principal subtypes are identified by immunophenotyping and include T lymphocyte and B lymphocyte types. In addition, the B cell type can be divided into a precursor B cell type, as well. Once these features are determined, subtypes may be referred to as acute T lymphoblastic leukemia or acute precursor (or pre) B cell lymphoblastic leukemia. One example of a marker useful in categorizing ALL subtypes is the common ALL antigen, cALLa, also called CD 10.

Despite overall improvements in outcome, the prognosis for patients whose leukemic blast cells carry the *BCR-ABL* fusion (created by the t[9;22]) or *MLL* gene rearrangements (created by translocations involving 11q23) is poor, with event-free survival (EFS) estimates of only about 30%. In fact, until recently, allogeneic hematopoietic stem cell transplantation (HSCT) during first remission was believed to be the only curative treatment option for these two groups of patients.

Additional types of tumors can also be treated using the methods described herein, including, e.g., neuroblastomas, myelomas, prostate cancers, small cell lung cancer, and others.

### First-Line Treatments

In numerous embodiments of the present invention, liposome-encapsulated vinca alkaloids are used as a first-line treatment for cancer. In preferred embodiments, liposome-encapsulated vinca alkaloids are used to treat leukemia, particularly acute lymphoblastic leukemia (ALL). As used herein, "first-line treatment" refers to a primary treatment for a patient first presenting with a cancer, in contrast to a relapsed or refractory cancer.

In such embodiments, the liposome-encapsulated vinca alkaloids can be used alone or in combination with one or more additional therapeutic agents. In certain embodiments, an additional therapeutic agent is a steroid. In one embodiment, it is dexamethasone.

### Relapsed or Refractory Forms of the Diseases

The present methods can also be used to treat primary, relapsed, transformed, or refractory forms of cancer. Often, patients with relapsed cancers have undergone one or more treatments including chemotherapy, radiation therapy, bone marrow transplants, hormone therapy, surgery, and the like. Of the patients who respond to such treatments, they may exhibit stable disease, a partial response (*i.e.*, the tumor or a cancer marker level diminishes by at least 50%), or a complete response (*i.e.*, the tumor as well as markers become undetectable). In either of these scenarios, the cancer may subsequently reappear, signifying a relapse of the cancer.

In certain embodiments, the methods provided herein will be used to treat a patient that has undergone a single course of treatment for a cancer, has partially or completely responded to such treatment, and has subsequently suffered a relapse. In other embodiments, patients are treated who have undergone more than one course of treatment, have responded more than once, and have subsequently suffered more than one relapse. The previous course of treatment can include any anti-cancer treatment, including chemotherapy, radiation therapy, bone marrow transplant, *etc.*

In certain embodiments of the present invention, liposomal alkaloids are employed against "resistant" cancers, *i.e.,* cancers which have previously exhibited a complete response to a treatment, but which subsequently manifest a resistance to second or later course of treatment.

### Vinca and Other Alkaloids

The present invention can include the use of naturally occurring vincristine or any synthetic derivative of naturally occuring vincristine. All of the above compounds are well known to those of skill and are readily available from commercial sources, by synthesis, or by purification from natural sources.

The vinca alkaloid used in the present invention is vincristine. Vincristine, also known as leurocristine sulfate, 22-oxovincaleukoblastine, Kyocristine, vincosid, vincrex, oncovin, Vincasar PFS^{®}, or VCR, is commercially available from any of a number of sources, e.g., Pharmacia & Upjohn, Lilly, IGT, etc. It is often supplied as vincristine sulfate, e.g., as a 1 mg/mL solution.

The present invention includes the use of a single vinca alkaloid. In addition, the vinca alkaloid can be combined with other compounds or molecules, such as other anti-neoplastic agents. In certain embodiments, such combinations of vinca alkaloid and/or other compounds can be made prior to liposomal formulation, thereby creating a combination within a single liposome. In other embodiments, the liposome-encapsulated vinca alkaloid is formulated and subsequently combined with the other molecules, which can themselves be free-form or liposome-encapsulated.

Any of the therapeutic agents described herein, including the liposome-encapsulated alkaloid, can be subjected to pre-clinical testing in well known models of human diseases. *In vivo* models of human lymphoma include mice carrying the non-Hodgkin's B-cell line DoHH2 (Kluin-Nelemans HC, et al. (1991) Leukemia 5(3) 221-224), or mice carrying Daudi or Raji cell xenografts (*see*, for example Hudson, WA et al. (1998) Leukemia 12(12): 2029-2033). Many other oncological models can also be used and are known to those skilled in the art.

### Lipids

Any of a number of lipids can be used to prepare the liposomes of the present invention, including amphipathic, neutral, cationic, and anionic lipids. Such lipids can be used alone or in combination, and can also include bilayer stabilizing components such as polyamide oligomers (*see*, *e.g.*, U.S. Patent Application Serial No. 09/218,988, filed December 22, 1998), peptides, proteins, detergents, lipid-derivatives, such as PEG coupled to phosphatidylethanolamine and PEG conjugated to ceramides (*see*, U.S. Application Serial No. 08/485,608). In a preferred embodiment, cloaking agents, which reduce elimination of liposomes by the host immune system, can also be included, such as polyamide-oligomer conjugates, *e.g.*, ATTA-lipids, (*see*, U.S. Patent Application Serial Number 08/996,783) and PEG-lipid conjugates (*see*, U.S. Patent Application Serial Nos. 08/486,214, 08/316,407 and 08/485,608).

Any of a number of neutral lipids can be included, referring to any of a number of lipid species that exist either in an uncharged or neutral zwitterionic form at physiological pH, including diacylphosphatidylcholine, diacylphosphatidylethanolamine, ceramide, sphingomyelin, cephalin, cholesterol, cerebrosides, and diacylglycerols.

In on embodiment, the lipid used is sphingomyelin. In particular embodiments, the lipid comprises sphingomyelin and cholesterol. In such embodiments, the ratio of sphingomyelin to cholesterol is typically between about 75/25 (mol% sphingomyelin/mol% cholesterol) and about 50/50 (mol% sphingomyelin/mol% cholesterol), about 70/30 and 55/45 (mol% sphingomyelin/mol% cholesterol), or about 55/45 (mol% sphingomyelin/mol% cholesterol). Such ratios, may be altered, however, by the addition of other lipids into the present formulations.

Cationic lipids, which carry a net positive charge at physiological pH, can readily be incorporated into liposomes for use in the present invention. Such lipids include, but are not limited to, N,N-dioleyl-N,N-dimethylammonium chloride ("DODAC"); N-(2,3-dioleyloxy)propyl-N,N-N-triethylammonium chloride ("DOTMA"); N,N-distearyl-N,N-dimethylammonium bromide ("DDAB"); N-(2,3-dioleoyloxy)propyl)-N,N,N-trimethylammonium chloride ("DOTAP"); 3β-(N-(N',N'-dimethylaminoethane)-carbamoyl)cholesterol ("DC-Chol"), N-(1-(2,3-dioleyloxy)propyl)-N-2-(sperminecarboxamido)ethyl)-N,N-dimethylammonium trifluoracetate ("DOSPA"), dioctadecylamidoglycyl carboxyspermine ("DOGS"), 1,2-dileoyl-sn-3-phosphoethanolamine ("DOPE"); and N-(1,2-dimyristyloxyprop-3-yl)-N,N-dimethyl-N-hydroxyethyl ammonium bromide ("DMRIE"). Additionally, a number of commercial preparations of cationic lipids can be used, such as LIPOFECTIN (including DOTMA and DOPE, available from GIBCO/BRL), LIPOFECTAMINE (comprising DOSPA and DOPE, available from GIBCO/BRL), and TRANSFECTAM (comprising DOGS, in ethanol, from Promega Corp.).

Anionic lipids suitable for use in the present invention include, but are not limited to, phosphatidylglycerol, cardiolipin, diacylphosphatidylserine, diacylphosphatidic acid, N-dodecanoyl phosphatidylethanoloamine, N-succinyl phosphatidylethanolamine, N-glutaryl phosphatidylethanolamine, lysylphosphatidylglycerol, and other anionic modifying groups joined to neutral lipids.

In numerous embodiments, amphipathic lipids are used. "Amphipathic lipids" refer to any suitable material, wherein the hydrophobic portion of the lipid material orients into a hydrophobic phase, while the hydrophilic portion orients toward the aqueous phase. Such compounds include, but are not limited to, phospholipids, aminolipids, and sphingolipids. Representative phospholipids include sphingomyelin, phosphatidylcholine, phosphatidylethanolamine, phosphatidylserine, phosphatidylinositol, phosphatidic acid, palmitoyloleoyl phosphatdylcholine, lysophosphatidylcholine, lysophosphatidylethanolamine, dipalmitoylphosphatidylcholine, dioleoylphosphatidylcholine, distearoylphosphatidylcholine, or dilinoleoylphosphatidylcholine. Other phosphorus-lacking compounds, such as sphingolipids, glycosphingolipid families, diacylglycerols, and β-acyloxyacids, can also be used. Additionally, such amphipathic lipids can be readily mixed with other lipids, such as triglycerides and sterols.

The liposomes used in the present invention can be multilamellar or unilamellar, which can be formed using the methods disclosed herein and other methods known to those of skill in the art.

Also suitable for inclusion in the present invention are programmable fusion lipid formulations. Such formulations have little tendency to fuse with cell membranes and deliver their payload until a given signal event occurs. This allows the lipid formulation to distribute more evenly after injection into an organism or disease site before it starts fusing with cells. The signal event can be, for example, a change in pH, temperature, ionic environment, or time. In the latter case, a fusion delaying or "cloaking" component, such as an ATTA-lipid conjugate or a PEG-lipid conjugate, can simply exchange out of the liposome membrane over time. By the time the formulation is suitably distributed in the body, it has lost sufficient cloaking agent so as to be fusogenic. With other signal events, its is desirable to choose a signal that is associated with the disease site or target cell, such as increased temperature at a site of inflammation.

### Preparation of Liposomes

A variety of methods are available for preparing liposomes as described in, e.g., Szoka, et al., Ann. Rev. Biophys. Bioeng., 9:467 (1980), U.S. Pat. Nos. 4,186,183, 4,217,344, 4,235,871, 4,261,975, 4,485,054, 4,501,728, 4,774,085, 4,837,028, 4,946,787, PCT Publication No. WO 91/17424, Deamer and Bangham, Biochim. Biophys. Acta, 443:629-634 (1976); Fraley, et al., Proc. Natl. Acad. Sci. USA, 76:3348-3352 (1979); Hope, et al., Biochim. Biophys. Acta, 812:55-65 (1985); Mayer, et al., Biochim. Biophys. Acta, 858:161-168 (1986); Williams, et al., Proc. Natl. Acad. Sci., 85:242-246 (1988), the text Liposomes, Marc J. Ostro, ed., Marcel Dekker, Inc., New York, 1983, Chapter 1, and Hope, et al., Chem. Phys. Lip., 40:89 (1986), all of which are incorporated herein by reference. Suitable methods include, but are not limited to, sonication, extrusion, high pressure/homogenization, microfluidization, detergent dialysis, calcium-induced fusion of small liposome vesicles, and ether-infusion methods.

One method produces multilamellar vesicles of heterogeneous sizes. In this method, the vesicle-forming lipids are dissolved in a suitable organic solvent or solvent system and dried under vacuum or an inert gas to form a thin lipid film. If desired, the film may be redissolved in a suitable solvent, such as tertiary butanol, and then lyophilized to form a more homogeneous lipid mixture, which is in a more easily hydrated powder-like form. This film is covered with an aqueous buffered solution and allowed to hydrate, typically over a 15-60 minute period with agitation. The size distribution of the resulting multilamellar vesicles can be shifted toward smaller sizes by hydrating the lipids under more vigorous agitation conditions or by adding solubilizing detergents, such as deoxycholate.

Unilamellar vesicles can be prepared by sonication or extrusion. Sonication is generally performed with a tip sonifier, such as a Branson tip sonifier, in an ice bath. Typically, the suspension is subjected to severed sonication cycles. Extrusion may be carried out by biomembrane extruders, such as the Lipex Biomembrane Extruder. Defined pore size in the extrusion filters may generate unilamellar liposomal vesicles of specific sizes. The liposomes may also be formed by extrusion through an asymmetric ceramic filter, such as a Ceraflow Microfilter, commercially available from the Norton Company, Worcester MA. Unilamellar vesicles can also be made by dissolving phospholipids in ethanol and then injecting the lipids into a buffer, causing the lipids to spontaneously form unilamellar vesicles. Also, phospholipids can be solubilized into a detergent, *e.g.*, cholates, Triton X, or n-alkylglucosides. Following the addition of the drug to the solubilized lipid-detergent micelles, the detergent is removed by any of a number of possible methods including dialysis, gel filtration, affinity chromatography, centrifugation, and ultrafiltration.

Following liposome preparation, the liposomes which have not been sized during formation may be sized to achieve a desired size range and relatively narrow distribution of liposome sizes. A size range of about 0.2-0.4 microns allows the liposome suspension to be sterilized by filtration through a conventional filter. The filter sterilization method can be carried out on a high through-put basis if the liposomes have been sized down to about 0.2-0.4 microns.

Several techniques are available for sizing liposomes to a desired size. One sizing method is described in U.S. Patent No. 4,737,323. Sonicating a liposome suspension either by bath or probe sonication produces a progressive size reduction down to small unilamellar vesicles less than about 0.05 microns in size. Homogenization is another method that relies on shearing energy to fragment large liposomes into smaller ones. In a typical homogenization procedure, multilamellar vesicles are recirculated through a standard emulsion homogenizer until selected liposome sizes, typically between about 0.1 and 0.5 microns, are observed. The size of the liposomal vesicles may be determined by quasi-electric light scattering (QELS), as described in Bloomfield, Ann. Rev. Biophys. Bioeng., 10:421-450 (1981). Average liposome diameter may be reduced by sonication of formed liposomes. Intermittent sonication cycles may be alternated with QELS assessment to guide efficient liposome synthesis.

Extrusion of liposome through a small-pore polycarbonate membrane or an asymmetric ceramic membrane is also an effective method for reducing liposome sizes to a relatively well-defined size distribution. Typically, the suspension is cycled through the membrane one or more times until the desired liposome size distribution is achieved. The liposomes may be extruded through successively smaller-pore membranes, to achieve gradual reduction in liposome size. In certain embodiments of the present invention, liposomes have a size ranging from about 0.05 microns to about 0.40 microns. In particular embodiments, liposomes are between about 0.05 and about 0.2 microns.

Empty liposomes may be prepared using any conventional method known to those of skill in the art.

Typically, as discussed *infra*, the liposomes used in the present invention will comprise a transmembrane potential, whereby antineoplastic agents such as vinca alkaloids are effectively loaded into and retained by the liposome. In particular embodiments, the potential will be effected by creating a pH gradient across the membrane. In one embodiment, the pH is lower at the interior of the liposomes than at the exterior. Such gradients can be achieved, *e.g.*, by formulating the liposomes in the presence of a buffer with a low pH, *e.g.*, having a pH between about 2 and about 6, and subsequently transferring the liposomes to a higher pH solution. In certain embodiments, the pH is between about 3 and 5, and in particular embodiments, the pH is about 4. Any of a number of buffers can be used, such as citrate.

Subsequently, before or after sizing, the external pH can be raised, *e.g.*, to about 7 or 7.5, by the addition of a suitable buffer, such as a sodium phosphate buffer. Raising the external pH creates a pH gradient across the liposomal membrane, thereby promoting efficient drug loading and retention.

Liposomes prepared according to these methods can be stored for substantial periods of time prior to drug loading and administration to a patient. For example, liposomes can be dehydrated, stored, and subsequently rehydrated, loaded with one or more vinca alkaloids, and administered. Dehydration can be accomplished, *e.g.*, using standard freeze-drying apparatus, *i.e.*, they are dehydrated under low pressure conditions. Also, the liposomes can be frozen, *e.g.*, in liquid nitrogen, prior to dehydration. Sugars can be added to the liposomal environment, *e.g.*, to the buffer containing the liposomes, prior to dehydration, thereby promoting the integrity of the liposome during dehydration. See, *e.g.*, U.S. Patent Nos. 5,077,056 and 5,736,155.

In numerous embodiments, the empty liposomes are first formulated in low pH buffer, and then manipulated in one of a variety of ways to obtain liposomes of the desired size. Methods for sizing liposomes include sonication, by bath or by probe, or homogenization. In particular embodiments, following such treatments, the liposomes are between about 0.05 to 0.45 microns. In certain embodiments, the liposomes are between about 0.05 and about 0.2 microns. Such sized liposomes can then be sterilized by filtration. Also, particle size distribution can be monitored by conventional laser-beam particle size discrimination or the like. In addition, methods of reducing liposome sizes to a relatively well defined size distribution are known, *e.g.*, one or more cycles of extrusion of the liposomes through a small-pore polycarbonate membrane or an asymmetric ceramic membrane.

### Preparation of Liposome-Encapsulated Vinca Alkaloid

Any of a number of methods can be used to load the vinca alkaloid and/or other drugs into the liposomes. Such methods include, *e.g.*, various encapsulation techniques and a transmembrane potential loading method. Generally, following such methods, the vinca alkaloids are present in the liposome at about 0.1 mg/mL to about 0.5 mg/mL. In certain embodiments, the vinca alkaloids are present at about 0.15 to 0.2 mg/mL.

In one encapsulation technique, the drug and liposome components are dissolved in an organic solvent in which all species are miscible and concentrated to a dry film. A buffer is then added to the dried film and liposomes are formed having the drug incorporated into the vesicle walls. Alternatively, the drug can be placed into a buffer and added to a dried film of only lipid components. In this manner, the drug will become encapsulated in the aqueous interior of the liposome. The buffer used in the formation of the liposomes can be any biologically compatible buffer solution of, for example, isotonic saline, phosphate buffered saline, or other low ionic strength buffers. The resulting liposomes encompassing the vinca alkaloids can then be sized as described above.

Transmembrane potential loading has been described in detail in U.S. Patent Nos. 4,885,172; 5,059,421; 5,171,578; and 5,837,282 (which teaches ionophore loading). Briefly, the transmembrane potential loading method can be used with essentially any conventional drug that can exist in a charged state when dissolved in an appropriate aqueous medium. Preferably, the drug will be relatively lipophilic so that it will partition into the liposome membranes. A transmembrane potential is created across the bilayers of the liposomes or protein-liposome complexes and the drug is loaded into the liposome by means of the transmembrane potential. The transmembrane potential is generated by creating a concentration gradient for one or more charged species (*e.g.*, Na⁺, K⁺, and/or H⁺) across the membranes. This concentration gradient is generated by producing liposomes having different internal and external media and has an associated proton gradient. Drug accumulation can then occur in a manner predicted by the Henderson-Hasselbach equation.

Descriptions of certain methods of preparing liposome-encapsulated vinca alkaloids for use in the present invention are discussed, *e.g.*, in U.S. Patent Nos. 5,741,516, 5,814,335 and 5,543,152. In one embodiment, liposomal vinca alkaloids are prepared prior to use from a kit including three or more vials. At least one of the vials contains a vincristine solution containing, *e.g.*, 1 mg/mL, 2 mg/mL, or 5 mg/mL vincristine sulfate in buffer containing, *e.g.*, 100 or 200 mg/mL mannitol (obtainable from, *e.g.*, SP Pharmaceuticals LLC, Albuquerque, NM; other excipients that are pharmaceutically acceptable, and in which vincristine remains stable for extended periods, can also be used) and sodium acetate adjusted to pH 3.5 to 5.5, or pH 4.5 to pH 4.7. One of the vials contains a solution containing liposomes comprising sphingomyelin and cholesterol (each of which is commercially available, *e.g.*, from NEN Life Sciences, Avanti Polar Lipids, *etc.*) and suspended in a 300 mM citrate buffer at, *e.g.*, pH 4.0. Another vial or vials contains a alkaline phosphate buffer (*e.g.*, pH 9.0) such as dibasic sodium phosphate, 14.2 mg/ml (20 ml/vial).

In preferred embodiments, a kit is used that contains two vials containing components that can be used to formulate the claimed liposome-encapsulated vincristine, or a kit containing one vial containing a stable preparation of liposomes comprising pre-loaded vincristine. Such stable preparations can be accomplished in any of a number of ways, including, but not limited to, (1) a hydrated preparation stored at ambient temperatures or refrigerated and which contains one or more modifications or components to enhance chemical stability, *e.g.*, antioxidants; (2) a hydrated preparation that was frozen and which includes a suitable excipient to protect from freeze/thaw-induced damage; or (3) a lyophilized preparation. Typically, any of the above-described kits also contain instructions for use, and may further comprise cleanup disposal materials.

To prepare the liposomes, the vincristine sulfate and liposome solutions are each added to a sterile vial and mixed, at an appropriate concentration ratio, *e.g.*, 0.01/1.0 to 0.2/1.0 (wt. vinca alkaloid/wt. lipid). The mixture is mixed, *e.g.*, by inverting the vial multiple times. Following the formation of the liposomes in low pH buffer, and either before or after the sizing of the liposomes, the liposomes are introduced into buffer of a higher pH, *e.g.*, a sodium phosphate buffer, thereby creating a pH gradient across the liposome surface. In certain embodiments, the external environment of the liposomes is between about pH 7.0 and about pH 7.5. The liposomes and vinca alkaloids can be mixed for an amount of time sufficient to achieve the desired alkaloid/lipid ratio. The mixture can be mixed, *e.g.*, by multiple inversions, and heated to temperatures between about 55°C and about 80°C, or between about 60°C and about 65°C, for about 5, 10, or more minutes. Such treatment causes greater than about 90% of the vincristine to become entrapped within the liposome.

In other embodiments, these steps are followed at a larger scale, and loaded liposomal vincristine is supplied to, *e.g.*, a hospital pharmacy in ready-to-administer format. Such larger scale formulations may be prepared from different starting materials than those described for the kit; in particular, the buffers may be different.

### Targeting Liposomes

In certain embodiments, it is desirable to target the liposomes of this invention using targeting moieties that are specific to a cell type or tissue. Targeting of liposomes using a variety of targeting moieties, such as ligands, cell surface receptors, glycoproteins, vitamins (*e.g.*, riboflavin) and monoclonal antibodies, has been previously described (*see*, *e.g.*, U.S. Patent Nos. 4,957,773 and 4,603,044. The targeting moieties can comprise the entire protein or fragments thereof.

Targeting mechanisms generally require that the targeting agents be positioned on the surface of the liposome in such a manner that the target moiety is available for interaction with the target, for example, a cell surface receptor. The liposome is designed to incorporate a connector portion into the membrane at the time of liposome formation. The connector portion must have a lipophilic portion that is firmly embedded and anchored into the membrane. It must also have a hydrophilic portion that is chemically available on the aqueous surface of the liposome. The hydrophilic portion is selected so as to be chemically suitable with the targeting agent, such that the portion and agent form a stable chemical bond. Therefore, the connector portion usually extends out from the liposomal surface and is configured to correctly position the targeting agent. In some cases, it is possible to attach the target agent directly to the connector portion, but in many instances, it is more suitable to use a third molecule to act as a "molecular bridge." The bridge links the connector portion and the target agent off of the surface of the liposome, thereby making the target agent freely available for interaction with the cellular target.

Standard methods for coupling the target agents can be used. For example, phosphatidylethanolamine, which can be activated for attachment of target agents, or derivatized lipophilic compounds, such as lipid-derivatized bleomycin, can be used. Antibody-targeted liposomes can be constructed using, for instance, liposomes that incorporate protein A (see, Renneisen, et al., J. Bio. Chem., 265:16337-16342 (1990) and Leonetti, et al., Proc. Natl. Acad. Sci. (USA), 87:2448-2451 (1990)). Other examples of antibody conjugation are disclosed in U.S. Patent Application Number 08/316,394. Examples of targeting moieties can also include other proteins, specific to cellular components, including antigens associated with neoplasms or tumors. Proteins used as targeting moieties can be attached to the liposomes via covalent bonds (see, Heath, Covalent Attachment of Proteins to Liposomes, 149 Methods in Enzymology 111-119 (Academic Press, Inc. 1987)). Other targeting methods include the biotin-avidin system.

### Administration of Lipid-Encapsulated Vinca Alkaloids

The liposome-encapsulated vinca alkaloid can be administered in any of a number of ways, including parenteral, intravenous, systemic, local, intratumoral, intramuscular, subcutaneous, intraperitoneal, inhalation, or any such method of delivery. In certain embodiments, the pharmaceutical compositions are administered intravenously by injection. In one embodiment, a patient is given an intravenous infusion of the liposome-encapsulated vinca alkaloid (*e.g.*, single agent) through a running intravenous line over, *e.g.*, 30 minutes, 60 minutes, 90 minutes, or longer. In one embodiment, a 60 minute infusion is used. Such infusions can be given periodically, *e.g.*, once every 1, 3, 5, 7, 10, 14, 21, or 28 days or longer. In certain embodiments, infusions are given every 7-21 days, and in particular embodiments, once every 7 days or once every 14 days. As used herein, each administration of a liposomal vinca alkaloid is considered one "course" of treatment.

Suitable formulation for use in the present invention can be found, *e.g.*, in Remington's Pharmaceutical Sciences, Mack Publishing Company, Philadelphia, PA, 17th Ed. (1985). Often, intravenous compositions will comprise a solution of the liposomes suspended in an acceptable carrier, such as an aqueous carrier. Any of a variety of aqueous carriers can be used, *e.g.*, water, buffered water, 0.4% saline, 0.9% isotonic saline, 0.3% glycine, 5% dextrose, and the like, and may include glycoproteins for enhanced stability, such as albumin, lipoprotein, globulin, *etc.* Often, normal buffered saline (135-150 mM NaCl) will be used. The compositions may contain pharmaceutically acceptable auxiliary substances as required to approximate physiological conditions, such as pH adjusting and buffering agents, tonicity adjusting agents, wetting agents, and the like, *e.g.*, sodium acetate, sodium lactate, sodium chloride, potassium chloride, calcium chloride, sorbitan monolaurate, triethanolamine oleate, *etc.* These compositions can be sterilized by conventional sterilization techniques, such as filtration or can be produced under sterile conditions. The concentration of liposomes in the carrier can vary. Generally, the concentration will be about 20-200 mg/mL; however persons of skill can vary the concentration to optimize treatment with different liposome components or for particular patients. For example, the concentration may be increased to lower the fluid load associated with treatment.

The amount of vinca alkaloid administered per dose is selected to be above the minimal therapeutic dose but below a toxic dose. The choice of amount per dose will depend on a number of factors, such as the medical history of the patient, the use of other therapies, and the nature of the disease. In certain embodiments, an initially low dose will be given, which can be increased based on the response and/or tolerance of the patient to the initial dose. In particular embodiments, the vinca alkaloid is administered at a dosage of at least 0.5, at least 1.0, at least 1.1, at least 1.2, at least 1.3, at least 1.4, at least 1.5, at least 1.6, at least 1.7, at least 1.8, at least 1.9, at least 2.0, at least 2.5, or at least 3.0 mg/m² (*i.e.*, mg vinca alkaloid vincristine per m² body surface area). In related embodiments, the vinca alkaloid vincristine is administered at a dose of about 1.4 to 2.4 mg/m² or about 1.4 to 2.8 mg/m². In particular embodiments a dose is 1.4 to 2.4 mg/m² or 1.4 to 2.8 mg/m². For example, 0.5, 1.0, 1.5, 2.0, 2.4, 2.8 mg/m² (mg vinca alkaloid vincristine per m² body surface area) or higher concentrations are administered, in certain embodiments. In one particular embodiment, patients are administered a dose of 2.4 mg/m², corresponding to a lipid dose of about 48 mg/m² or about 1.3 mg/kg lipid and 0.06 mg/kg vincristine for an average 70 kg patient, or about 3 mg to about 6 mg vincristine per dose. In another particular embodiment, patients are administered a dose of 2.0 mg/m², corresponding to a lipid dose of about 40 mg/m² or about 1.1 mg/kg lipid and 0.05 mg/kg vincristine for an average 70 kg patient, or about 3 mg to about 6 mg vincristine per dose.

Patients typically will receive at least two courses of such treatment, and potentially more, depending on the response of the patient to the treatment. In single agent regimens, total courses of treatment are determined by the patient and physician based on observed responses and toxicity. Similarly, the number of courses of treatment using liposome-encapsulated vincristine in combination with dexamethasone will be determined by the patient and physician.

Because vincristine dosages are limited by neurotoxicity in humans, it is sometimes useful to co-administer liposomal vincristine with a treatment for neurotoxicity. This treatment may be prophylactic or therapeutic. An example is the administration of gabapentin Neurontin™ (Parke-Davis), or neurotonin, for treatment of neuropathic pain, *e.g.*, 100-200 mg Neurontin™ is administered 3 times per day to an adult patient. If neuropathic pain improves, then liposomal vincristine treatments may continue. Because this type of prophylactic or therapeutic treatment is intended only to treat side-effects of liposomal vincristine, it is considered separately from the combination therapies set forth below. The methods and dosages of the present invention are associated with reduced toxicity *e.g.* cardiotoxicity, as compared to the use of fre vincristine.

This invention is based, in part, on the surprising discovery that, in contrast to free form vinca alkaloids, a liposome-encapsulated vinca alkaloid can be administered without a cap on the total dosage. For example, whereas free form vincristine is typically administered with a cap of 2.0 or 2.5 mg, liposome-encapsulated vincristine can be administered at constant dosages of, for example, 2.0 mg/m² or 2.4 mg/m² without a cap of 2.0 or 2.5 mg total dose. Accordingly, in particular embodiments, liposomal vinca alkaloid vincristine is administered at a constant dosage without a dose cap. In particular embodiments, the constant dosage is at least 0.5, at least 1.0, at least 1.1, at least 1.2, at least 1.3, at least 1.4, at least 1.5, at least 1.6, at least 1.7, at least 1.8, at least 1.9, at least 2.0, at least 2.5, or at least 3.0 mg/m². Thus, in particular embodiments, for a typical patient of from 1.5 to 3.0 m² surface area, doses of from about 3.6 to about 7.2 mg vincristine or from about 3.0 to about 6.0 mg vincristine are administered.

### Combination Therapies

Since different classes of chemotherapeutic agents generally have different mechanisms of action, combinations of chemotherapeutic agents are often used in an effort to attack tumor cells through multiple mechanisms and thereby more effectively halt tumor growth and kill tumor cells. Combination therapy using multiple classes of chemotherapeutic agents is also used to avoid cross-resistance to drugs. For example, taxanes have been used in combination with a variety of other antitumor drugs, including, *e.g.*, the cyclin-dependent kinase inhibitor, flavopiridol, the platinum-based drug, carboplatin, the peptidomimetic inhibitor of famesyl transferase, ER-51785, the EGFR-selective tyrosine kinase inhibitor, IRESSA (ZD1839), cyclosporine, and trastuzumab. Schwartz, G.K., et al. J. Clin. Oncol. 20(8):2157-70 (2002), Ogawara, M., et al., Jpn J. Clin. Oncol. 32(2):48-53 (2002), Nakamura, K., et al., Oncol. Res. 12(11-12):477-84 (2001), Ciardiello, F. et al., Int. J. Cancer 98(3):463-9 (2002), Chiou, W.L., et al., J. Clin. Oncol. 20(7):1951-2 (2002), and Esteva, F.J., et al., J. Clin. Oncol. 20(7):1800-8 (2002).

Combination drug therapy is frequently limited due to toxic side effects associated with the combination of drugs. These undesirable side effects may be associated with either the drug or its delivery vehicle. For example, the use of paclitaxel in combination with other chemotherapeutic agents is limited by the acute lethal toxicity of the Cremophor vehicle and paclitaxel-associated neutropenia. Such side effects pose particular problems for combination therapy when the drugs cause similar side effects or contain the same toxic vehicle. In such circumstances, it is frequently not possible to administer each drug at the dosage found to be the most effective when used alone. Accordingly, suboptimal doses may be used, with the effectiveness of each drug compromised.

Liposome-encapsulated vinca alkaloid is administered in combination with one or more additional compounds or therapies. For example, the vinca alkaloid can be administered in conjunction with another therapeutic compound, such as cyclophosphamide, prednisone, other alkaloids such as the taxanes, camptothecins, and/or podophyllins, and/or other chemotherapeutic agents such as antisense drugs or anti-tumor vaccines. Liposome-encapsulated vincristine is co-administered with dexamethasone. In certain embodiments, multiple compounds are loaded into the same liposomes. In other embodiments, the liposome-encapsulated vinca alkaloid is formed individually and subsequently combined with other compounds for a single coadministration. Alternatively, certain therapies are administered sequentially in a predetermined order, such as in CHOP or lipo-CHOP.

Liposome-encapsulated vincristine is administered in combination with another therapeutic agent, such as dexamethasone, without further administration of an anthracycline.

The liposome-encapsulated vinca alkaloid can also be combined with anti-tumor agents such as monoclonal antibodies including, but not limited to, Oncolym™ (Techniclone Corp. Tustin, CA) or Rituxan™ (IDEC Pharmaceuticals), Bexxar™ (Coulter Pharmaceuticals, Palo Alto, CA), or IDEC-Y2B8 (IDEC Pharmaceuticals Corporation). In addition, liposome-encapsulated vinca alkaloid can be administered along with one or more non-molecular treatments such as radiation therapy, bone marrow transplantation, hormone therapy, surgery, *etc.*

In one embodiment, the liposome-encapsulated vinca alkaloid is administered in combination with an anti-cancer compound or therapy that provides an increased or synergistic improvement in tumor reduction based on mechanism of action and non-overlapping toxicity profiles. In particular embodiments, the liposomal vinca alkaloid can be delivered with a taxane, which optionally may also be a liposomal taxane. While it is thought that vinca alkaloids depolymerize microtubules and taxanes stabilize microtubules, the two compounds have been found to act synergistically in the impairment of tumor growth, presumably because both are involved in the inhibition of microtubule dynamics. *See*, Dumontet, C. and Sikic, B.I., (1999) J. Clin Onc. 17(3) 1061-1070. Liposomal formulations of the vinca alkaloid according to the present invention will thus significantly diminish the myeloid and neurologic toxicity associated with the sequential administration of free form vinca alkaloids and taxanes.

Liposome-encapsulated vinca alkaloids may be delivered with one or more additional chemotherapeutic agents. In certain embodiments, the liposome-encapsulated drug and the other treatment or drug used in combination have different mechanisms of action, and they may act additively, cooperatively or synergistically to combat a disease. In other embodiments, the liposome-encapsulated drug and the other treatment or drug used in combination have the same or similar mechanisms of action, and they may also act additively, cooperatively or synergistically to combat a disease. For example, drugs that are active during S phase of the cell cycle may be used in combination with drugs that are active during M-phase.

Chemotherapeutic drugs may be classified into a large number of groups, based upon their mechanism of action, including, for example, platinates, alkylating agents, antimetabolites, plant alkaloids, antimicrotubule agents, antibiotics, steroids, hormonal agents, interleukins, mitotic inhibitors, angiogenesis inhibitors, apoptosis promoters, and biological response modifiers. Typically, each of these classes of drugs acts to inhibit tumor cell growth or proliferation via a different molecular mechanism. For example, selective estrogen-receptor modulators bind to estrogen receptors of estrogen-dependent breast cancer cells and prevent estrogen binding, thereby effectively starving these cancer cells. In completely different modes of action, nucleoside analogs, such as azacytidine and flurouracil, inhibit nucleic acid synthesis and metabolism. In particular embodiments the liposome-encapsulated vinca alkaloid is delivered in combination with another drug of the same class, while in other embodiments, the liposome-encapsulated vinca alkaloid is administered with drugs of a different class. Since different classes of chemotherapeutic agents generally have different mechanisms of action, combinations of chemotherapeutic agents are often used in an effort to attack tumor cells through multiple mechanisms and thereby more effectively halt tumor growth and kill tumor cells. Combination therapy using multiple classes of chemotherapeutic agents is also used to avoid cross-resistance to drugs.

In particular embodiments, the liposome-encapsulated vinca alkaloid vincristine is administered in combination with another therapy used for the treatment of ALL. A variety of therapies are used for the treatment of ALL, including chemotherapy, radiation therapy, and stem cell transplant.

When used for the treatment of ALL, chemotherapeutic agents are generally delivered by one or more of several different means. Chemotherapeutic agents may be delivered systemically, either by mouth or injection into a vein or muscle. A chemotherapeutic agent may also be delivered intrathecally, directly in the spinal column (intrathecal), a body cavity or an organ, so that the drug mainly affect cancer cells in those areas. Intrathecal chemotherapy is frequently used to treat ALL that has spread, or may spread, to the brain and spinal cord. When used to prevent cancer from spreading to the brain and spinal cord, it is called central nervous system (CNS) sanctuary therapy or CNS prophylaxis. Intrathecal chemotherapy is often given in addition to chemotherapy by mouth or vein.

Examples of specific chemotherapeutic agents that may be administered in combination with the liposome-encapsulated vinca alkaloid vincristine according to the present invention include, but are not limited to, dexamethasone, fludarabine, cyclophosphamide, imatinib, valspodar, asparaginase, cytarabine, dexrazoxane, hydrocortisone, leucovorin calcium, mercaptopurine, methotrexate, methylprednisolone, prednisolone, and prednisone.

Liposomal vincristine is administered in combination with dexamethasone.

Other combination therapies known to those of skill in the art can be used in conjunction with the methods of the present invention.

### EXAMPLES

The following examples are offered to illustrate, but not to limit the claimed invention.

### EXAMPLE 1

### MAKING LIPOSOME-ENCAPSULATED VINCRISTINE

Liposome-encapsulated vincristine (Vincristine Sulfate Liposome Injection) was prepared using a 5 vial kit. Vials 1 and 2 contained a vincristine sulfate solution (1 mg/mL Vincasar PFS^{®,} SP Pharmaceuticals LLC, Albuquerque, NM) in buffer comprising mannitol and sodium acetate, pH 4.5-4.7; vial 3 contained empty liposomes (100 mg/mL Sphingomyelin/Cholesterol liposomes, at a ratio of between about 60/40 to 50/50, or more preferably 58/42 mol%/mol%) in buffer comprising 300 mM citrate at pH 4.0; vial 4 contained an alkaline phosphate buffer (14.2 mg/mL dibasic sodium phosphate hepta hydrate); and vial 5 was an empty, sterile vial. The foregoing empty liposomes were prepared by dilution of an ethanol solution of sphingomyelin and cholesterol in citrate buffer to form large multilamellar vesicles. These were then extruded using standard techniques, as described in U.S. Patent No. 5,741,516, to form large unilamellar vesicles (diameter approximately 100-120 nm).

4 mL of vincristine sulfate solution was removed from vials 1 and 2 and added to sterile vial 5. Subsequently, 0.8 mL Sphingomyelin/Cholesterol liposomes was removed from vial 3 and added to vial 5. Vial 5 was inverted five times to mix the materials. 20 mL of the sodium phosphate solution from vial 4 was added to vial 5. Vial 5 was again inverted five times, without shaking, to mix the materials. Vial 5 was then heated in a water bath at 60-65°C for five minutes, after which the vial was again inverted five times. The vial was then again heated for five minutes and inverted five more times.

The final product (sphingosomal vincristine, SV) contained 0.16 mg/mL vincristine sulfate and 3.2 mg/mL total lipid.

### EXAMPLE 2

### TREATMENT OF RELAPSED OR REFRACTORY ACUTE LYMPHOCYTIC LEUKEMIA (ALL) WITH SPHINGOSOMAL VINCRISTINE (SV) AND DEXAMETHASONE

Vincristine and dexamethasone are generally used in the remission induction phase of ALL therapy, but must be combined with an anthracycline (doxorubicin or daunorubicin) to achieve a high complete response (CR) rate (Gottlieb et al., Blood 64:267-274, 1984). Unfortunately, however, this regimen often causes severe toxicity, including myelosuppression and cardiotoxicity. Given that many patients have compromised bone marrow function resulting from the expansion of leukemic blasts in the marrow, myelosuppressive combinations can induce severe neuropenia with attendant susceptibility to life-threatening infections. Further, particularly in the elderly, co-morbidities can significantly reduce the patient's ability to tolerate intensive chemotherapy. Inclusion of anthracyclines in the combination can also induce cardiotoxicities, further limiting such regimens in patients with underlying cardiac conditions.

The effect of using liposome-encapsulated vincristine in a combination with another chemotherapeutic agent for the treatment of relapsed or refractory ALL was examined, in order to determine whether the use of liposome-encapsulated vincristine would negate the need to also use an anthracycline (doxorubicin or daunorubicin) to achieve a high complete response (CR), thereby reducing the toxicities, including myelosuppression and cardiotoxicity, associated with anthracyclines, without compromising the overall therapeutic outcome.

A phase I clinical trial was undertaken in twenty patients diagnosed with relapsed or refractory ALL (including Burkitt cell ALL) without greater than grade 2 prior or active central or peripheral neuropathy (PN). The median age of the patients was 36 years (range, 21- 62). Thirty percent of the patients were refractory to induction therapy, and the median number of prior salvage regimens was two (range, 0 - 3).

Patients were treated with liposome-encapsulated vincristine (SV; as described above) weekly (dose escalated with three subjects in each cohort and expansion to six for toxicity) and pulse dexamethasone (40 mg daily days 1-4 and 11-14). One course of treatment was defined as four weekly doses of SV. Three patients were treated with weekly SV at 1.5 mg/m²; three patients were treated with weekly SV at 1.825 mg/m²; five patients were treated with weekly SV at 2.0 mg/m²; seven patients were treated with weekly SV at 2.25 mg/m²; and two patients were treated with weekly SV at 2.4 mg/m² (Table 1).

**Table 1. Dose Escalation Cohorts for Sphingosomal Vincristine**

| Vincristine Dose¹ | Dexamethasone Dose² | Number of Patients |
|---|---|---|
| 1.5 mg/m² | 40 mg | 3 |
| 1.825 mg/m² | 40 mg | 3 |
| 2.0 mg/m² | 40 mg | 5 |
| 2.25 mg/m² | 40 mg | 7 |
| 2.4 mg/m² | 40 mg | 2 |

| | | |
|---|---|---|
| ¹Weekly with no dose capping ²Days 1-4 and 11-14 | | |

Non-hematologic toxicities attributed to SV included grade 1-2 PN in nearly all patients and tumor lysis syndrome in one patient. Five patients had transient grade 3-4 elevations in hepatic transaminases attributed to azole antifungal prophylaxis. Grade 3 infections (*e.g.*, bacteremia or fungal processes) were related to baseline neutropenia in six patients and SV-induced neutropenia in four patients. No dose-limiting toxicities have been observed at the doses evaluated to date, and additional studies are performed using higher dosages.

The toxicity study results were surprising, since free vincristine is generally administered at 1.4 mg/m², often with dose capping at 2.0 mg or 2.5 mg total dose. Doses of SV were not capped and hence total vincristine doses of approximately 4.1 mg were administered for the highest dose cohort (based on an average BSA of 1.7 m²). Furthermore, these high doses were administered on a weekly schedule with only moderate peripheral neurotoxicity observed (grades 1-2).

Of the twenty evaluable patients, the following best responses were achieved (Table 2). Six patients (30%) achieved complete remission (CR), and one patient achieved a partial response. The overall response rate observed in this Phase I study was therefore 35%. This is an unexpectedly high response rate given that patients were either relapsed or refractory to prior therapy and given that many patients were treated at liposomal vincristine doses that are below the maximum tolerated dose. Of the remaining thirteen patients, two showed some hematological improvement. Two patients discontinued therapy early (one for PD after 3 doses of SV and one withdrew consent after 3 doses), and one patient had therapy interrupted after one dose of SV owing to *C. difficile* colitis. One CR patient relapsed after three months and achieved a third CR with hyper-CVAD followed by allogeneic stem cell transplant (SCT). Three patients went on to SCT while in CR (one died after sepsis).

**Table 2. Response Evaluation for Sphingosomal Vincristine and Dexamethasone in ALL by Vincristine Dose and Overall Response Rate**

| Vincristine Dose | Number of Patients | Number of Patients with Partial Response | Number of Patients with Complete Response |
|---|---|---|---|
| 1.5 mg/m² | 3 | - | 1 |
| 1.825 mg/m² | 3 | - | 1 |
| 2.0 mg/m² | 5 | 1 | 1 |
| 2.25 mg/m² | 7 | - | 2 |
| 2.4 mg/m² | 2 | - | 1 |
| | | | |
| All doses | 20 | 1 | 6 |

These results demonstrate that liposome-encapsulated vincristine in combination with dexamethasone is particularly efficacious in the treatment of relapsed and refractory ALL, and further establish that liposome-encapsulated vinca alkaloids are a superior alternative to free vinca alkaloids in the treatment of cancers, including relapsed and refractory leukemias and lymphomas. Liposome-encapsulated vincristine may be used at a higher dose than free vincristine, and does not require the combined use of an anthracycline. This greatly reduces associated toxicities, thereby allowing safer treatment for a larger number of patients.

## Claims

1. Use of liposome-encapsulated vincristine in combination with dexamethasone for the preparation of a medicament for the treatment of cancer in a human, wherein said cancer is a lymphoma or leukemia and wherein the treatment does not further comprise administering an anthracycline to said human.

2. The use of claim 1, wherein said liposome comprises sphingomyelin and cholesterol.

3. The use of claim 2, wherein the ratio of sphingomyelin to cholesterol is between 75/25 (mol% sphingomyelin/mol% cholesterol) and 50/50 (mol% sphingomyelin/mol% cholesterol).

4. The use of claim 3, wherein the ratio of sphingomyelin to cholesterol is about 55/45 (mol% sphingomyelin/mol% cholesterol).

5. The use of claim 1, wherein said vincristine is for administration at a dosage of between 1.4 - 2.8 mg/m²_{.}

6. The use of claim 5, wherein said vincristine is for administration at a dosage of between 1.4 - 2.4 mg/m².

7. The use of claim 1, wherein said vincristine is for administration at a dosage greater than 1.4 mg/m².

8. The use of claim 1, wherein said dexamethasone is for administration at a dosage of between 25-75 mg.

9. The use of claim 1, wherein said leukemia is Acute Lymphoblastic Leukemia (ALL).

10. The use of claim 1, wherein said treatment is a first-line treatment.

11. The use of claim 1, wherein said cancer is a relapsed or refractory cancer.

12. Use of liposome-encapsulated vincristine in combination with dexamethasone for the preparation of a medicament for the treatment of Acute Lymphoblastic Leukemia (ALL) in a human, wherein the treatment does not further comprise administering an anthracycline to said human.

13. The use of claim 12, wherein said treatment is a first- line treatment.

14. The use of claim 12, wherein said ALL is a relapsed ALL.

15. The use of claim 12, wherein said liposome comprises sphingomyelin and cholesterol.

16. The use of claim 15, wherein the ratio of sphingomyelin to cholesterol is between 75/25 (mol% sphingomyelin/mol% cholesterol) and 50/50 (mol% sphingomyelin/mol% cholesterol).

17. The use of claim 16, wherein the ratio of sphingomyelin to cholesterol is 55/45 (mol% sphingomyelin/mol% cholesterol).

18. The use of claim 12, wherein said vincristine is for administration at a dosage of between 1.4 - 2.8 mg/m²_{.}

19. The use of claim 18, wherein said vincristine is for administration at a dosage of between 1.4 - 2.4 mg/m².

20. The use of claim 12, wherein said vincristine is for administration at a dosage greater than 1.4 mg/m².

21. The use of claim 12, wherein said dexamethasone is for administration at a dosage of between at 25-75 mg.

22. The use of claim 12, wherein said vincristine is for administration at a dosage greater than 1.4 mg/m², said dexamethasone is for administration at a dosage of between 25-75 mg.

23. A combination of liposome-encapsulated vincristine and dexamethasone for use in the treatment of cancer in a human, wherein said cancer is a lymphoma or leukemia and wherein the treatment does not further comprise administering an anthracycline to said human.

24. A combination of liposome-encapsulated vincristine and dexamethasone for use in the treatment of Acute Lymphoblastic Leukemia (ALL) in a human, wherein the treatment does not further comprise administering an anthracycline to said human.

## Patentansprüche

1. Verwendung von Liposom-verkapseltem Vincristin in Kombination mit Dexamethason für die Herstellung eines Medikaments für die Behandlung von Krebs bei einem Menschen, wobei der Krebs ein Lymphom oder Leukämie ist und wobei die Behandlung nicht zusätzlich das Verabreichen eines Anthracyclins an den Menschen umfasst.

2. Verwendung nach Anspruch 1, wobei das Liposom Sphingomyelin und Cholesterin umfasst.

3. Verwendung nach Anspruch 2, wobei das Verhältnis von Sphingomyelin zu Cholesterin zwischen 75/25 (mol% Sphingomyelin / mol% Cholesterin) und 50/50 (mol% Sphingomyelin / mol% Cholesterin) beträgt.

4. Verwendung nach Anspruch 3, wobei das Verhältnis von Sphingomyelin zu Cholesterin ungefähr 55/45 (mol% Sphingomyelin / mol% Cholesterin) beträgt.

5. Verwendung nach Anspruch 1, wobei das Vincristin zur Verabreichung in einer Dosis zwischen 1,4 - 2,8 mg/m² bestimmt ist.

6. Verwendung nach Anspruch 5, wobei das Vincristin zur Verabreichung in einer Dosis zwischen 1,4 - 2,4 mg/m² bestimmt ist.

7. Verwendung nach Anspruch 1, wobei das Vincristin zur Verabreichung in einer Dosis von mehr als 1,4 mg/m² bestimmt ist.

8. Verwendung nach Anspruch 1, wobei das Dexamethason zur Verabreichung in einer Dosis zwischen 25 - 75 mg bestimmt ist.

9. Verwendung nach Anspruch 1, wobei die Leukämie akute lymphoblastische Leukämie (ALL) ist.

10. Verwendung nach Anspruch 1, wobei die Behandlung eine Primärbehandlung ist.

11. Verwendung nach Anspruch 1, wobei der Krebs ein rezidivierender oder refraktärer Krebs ist.

12. Verwendung von Liposom-verkapseltem Vincristin in Kombination mit Dexamethason für die Herstellung eines Medikaments für die Behandlung von akuter lymphoblastischer Leukämie (ALL) bei einem Menschen, wobei die Behandlung nicht zusätzlich das Verabreichen eines Anthracyclins an den Menschen umfasst.

13. Verwendung nach Anspruch 12, wobei die Behandlung eine Primärbehandlung ist.

14. Verwendung nach Anspruch 12, wobei die ALL eine rezidivierende ALL ist.

15. Verwendung nach Anspruch 12, wobei das Liposom Sphingomyelin und Cholesterin umfasst.

16. Verwendung nach Anspruch 15, wobei das Verhältnis von Sphingomyelin zu Cholesterin zwischen 75/25 (mol% Sphingomyelin / mol% Cholesterin) und 50/50 (mol% Sphingomyelin / mol% Cholesterin) beträgt.

17. Verwendung nach Anspruch 16, wobei das Verhältnis von Sphingomyelin zu Cholesterin 55/45 (mol% Sphingomyelin / mol% Cholesterin) beträgt.

18. Verwendung nach Anspruch 12, wobei das Vincristin zur Verabreichung in einer Dosis zwischen 1,4 - 2,8 mg/m² bestimmt ist.

19. Verwendung nach Anspruch 18, wobei das Vincristin zur Verabreichung in einer Dosis zwischen 1,4 - 2,4 mg/m² bestimmt ist.

20. Verwendung nach Anspruch 12, wobei das Vincristin zur Verabreichung in einer Dosis von mehr als 1,4 mg/m² bestimmt ist.

21. Verwendung nach Anspruch 12, wobei das Dexamethason zur Verabreichung in einer Dosis zwischen 25 - 75 mg bestimmt ist.

22. Verwendung nach Anspruch 12, wobei das Vincristin zur Verabreichung in einer Dosis von mehr als 1,4 mg/m² bestimmt ist und das Dexamethason zur Verabreichung in einer Dosis zwischen 25 - 75 mg bestimmt ist.

23. Kombination von Liposom-verkapseltem Vincristin und Dexamethason zur Verwendung bei der Behandlung von Krebs bei einem Menschen, wobei der Krebs ein Lymphom oder Leukämie ist und wobei die Behandlung nicht zusätzlich das Verabreichen eines Anthracyclins an den Menschen umfasst.

24. Kombination von Liposom-verkapseltem Vincristin und Dexamethason zur Verwendung bei der Behandlung von akuter lymphoblastischer Leukämie (ALL) bei einem Menschen, wobei die Behandlung nicht zusätzlich das Verabreichen eines Anthracyclins an den Menschen umfasst.

## Revendications

1. Utilisation de vincristine encapsulée dans un liposome en combinaison avec de la dexaméthasone pour la préparation d'un médicament destiné au traitement d'un cancer chez un humain, dans laquelle ledit cancer est un lymphome ou une leucémie et dans laquelle le traitement ne comprend pas l'administration supplémentaire d'une anthracycline audit humain.

2. Utilisation selon la revendication 1, dans laquelle ledit liposome comprend une sphingomyéline et du cholestérol.

3. Utilisation selon la revendication 2, dans laquelle le rapport de la sphingomyéline au cholestérol est compris entre 75/25 (% en moles de sphingomyéline / % en moles de cholestérol) et 50/50 (% en moles de sphingomyéline / % en moles de cholestérol).

4. Utilisation selon la revendication 3, dans laquelle le rapport de la sphingomyéline au cholestérol est d'environ 55/45 (% en moles de sphingomyéline / % en moles de cholestérol).

5. Utilisation selon la revendication 1, dans laquelle ladite vincristine est destinée à être administrée à une dose comprise entre 1,4 et 2,8 mg/m².

6. Utilisation selon la revendication 5, dans laquelle ladite vincristine est destinée à être administrée à une dose comprise entre 1,4 et 2,4 mg/m².

7. Utilisation selon la revendication 1, dans laquelle ladite vincristine est destinée à être administrée à une dose supérieure à 1,4 mg/m².

8. Utilisation selon la revendication 1, dans laquelle ladite dexaméthasone est destinée à être administrée à une dose comprise entre 25 et 75 mg.

9. Utilisation selon la revendication 1, dans laquelle ladite leucémie est une leucémie aiguë lymphoblastique (LAL).

10. Utilisation selon la revendication 1, dans laquelle ledit traitement est un traitement de première intention.

11. Utilisation selon la revendication 1, dans laquelle ledit cancer est un cancer récidivant ou réfractaire.

12. Utilisation de vincristine encapsulée dans un liposome en combinaison avec de la dexaméthasone pour la préparation d'un médicament destiné au traitement d'une leucémie aiguë lymphoblastique (LAL) chez un humain, dans laquelle le traitement ne comprend pas l'administration supplémentaire d'une anthracycline audit humain.

13. Utilisation selon la revendication 12, dans laquelle ledit traitement est un traitement de première intention.

14. Utilisation selon la revendication 12, dans laquelle ladite LAL est une LAL récidivante.

15. Utilisation selon la revendication 12, dans laquelle ledit liposome comprend une sphingomyéline et du cholestérol.

16. Utilisation selon la revendication 15, dans laquelle le rapport de la sphingomyéline au cholestérol est compris entre 75/25 (% en moles de sphingomyéline / % en moles de cholestérol) et 50/50 (% en moles de sphingomyéline / % en moles de cholestérol).

17. Utilisation selon la revendication 16, dans laquelle le rapport de la sphingomyéline au cholestérol est de 55/45 (% en moles de sphingomyéline / % en moles de cholestérol).

18. Utilisation selon la revendication 12, dans laquelle ladite vincristine est destinée à être administrée à une dose comprise entre 1,4 et 2,8 mg/m².

19. Utilisation selon la revendication 18, dans laquelle ladite vincristine est destinée à être administrée à une dose comprise entre 1,4 et 2,4 mg/m².

20. Utilisation selon la revendication 12, dans laquelle ladite vincristine est destinée à être administrée à une dose supérieure à 1,4 mg/m².

21. Utilisation selon la revendication 12, dans laquelle ladite dexaméthasone est destinée à être administrée à une dose comprise entre 25 et 75 mg.

22. Utilisation selon la revendication 12, dans laquelle ladite vincristine est destinée à être administrée à une dose supérieure à 1,4 mg/m² et ladite dexaméthasone est destinée à être administrée à une dose comprise entre 25 et 75 mg.

23. Combinaison de vincristine encapsulée dans un liposome et de dexaméthasone pour utilisation dans le traitement d'un cancer chez un humain, dans laquelle ledit cancer est un lymphome ou une leucémie et dans laquelle le traitement ne comprend pas l'administration supplémentaire d'une anthracycline audit humain.

24. Combinaison de vincristine encapsulée dans un liposome et de dexaméthasone pour utilisation dans le traitement d'une leucémie aiguë lymphoblastique (LAL) chez un humain, dans laquelle le traitement ne comprend pas l'administration supplémentaire d'une anthracycline audit humain.
